# EUROPÄISCHE PATENTANMELDUNG

(11) **EP 1 854 486 A2**
(43) Veröffentlichungstag der Anmeldung: **14.11.2007**
(21) Anmeldenummer: 07104021.6
(22) Anmeldetag: 13.03.2007
(51) Int. Cl.: A61K 45/06, A61K 36/82, A61K 36/48, A61K 36/41, A61K 33/32, A61K 33/30, A61K 33/24, A61K 33/06, A61K 33/04, A61K 31/714, A61K 31/685, A61K 31/445, A61K 31/4415, A61K 31/4172, A61K 31/405, A61K 31/375, A61K 31/355, A61K 31/198, A61K 31/197, A61K 31/185, A23L 1/305, A23L 1/304, A23L 1/302

(54) **Verwendung von Neurotransmitter-Derivaten zur Herstellung von Mitteln zur Behandlung von neuroendokrinen gesundheitlichen Störungen**

(30) Priorität: 14.03.2006 DE 102006012171; 04.08.2006 DE 102006038224
(71) Anmelder: Bieger, Wilfried P., 80336 München (DE)
(72) Erfinder: Bieger, Wilfried P., 80336 München (DE)
(74) Vertreter: Wehlan, Helmut

(57) **Zusammenfassung**

Die Erfindung betrifft die Verwendung von Neurotransmitter-Derivaten zur Herstellung von Mitteln zur Behandlung von neuroendokrinen gesundheitlichen Störungen. Die erfindungsgemäß eingesetzten Mittel und Nahrungsergänzungsmittel sind insbesondere zur Behandlung von Neurotransmitter- /Stresshormon-Ungleichgewichten des neuroendokrinen Regelkreises und damit einhergehender Erkrankungen geeignet. Sie enthalten als wirksame Komponenten Neurotransmitter-Derivate, wie Aminosäurevorstufen von Neurotransmittern, Kofaktoren für die Synthese von Neurotransmittern und Neurotransmitter-Modulatoren.

## Beschreibung

Die Erfindung betrifft die Verwendung von Neurotransmitter-Derivaten zur Herstellung von Mitteln, die zur Behandlung von neuroendokrinen gesundheitlichen Störungen bzw. mit Stresshormon- oder Neurotransmitter-Ungleichgewichten des neuroendokrinen Regelkreises einhergehenden Erkrankungen geeignet sind. Die erfindungsgemäß eingesetzten Mittel und Nahrungsergänzungsmittel enthalten als wirksame Komponenten Neurotransmitter-Derivate, wie Aminosäurevorstufen von Neurotransmittern, Kofaktoren für die Synthese von Neurotransmittern und Neurotransmitter-Modulatoren.

Neurotransmitter-Störungen beziehen sich sowohl auf Defizite als auch auf Überschüsse, also generell auf Neurotransmitter-Ungleichgewichte, wie z.B. bei Stresszuständen oder bei Depressionen, bei denen hohe Noradrenalin- und Cortisol-Werte neben niedrigen Serotonin-Gehalten festgestellt worden sind. Bei derartigen Erkrankungen sind diese Ungleichgewichte als die zentrale Störung auf der Ebene der Neurotransmitter anzusehen. Im Falle von Depressionen und bei einzelnen psychiatrischen Diagnosen sind Behandlungen mit L-Tryptophan, insbesondere mit 5-Hydroxytryptophan, publiziert worden (Alternative Medicine Review, Volume 3, Number 4, 1998, 224-226).

Bei Neurotransmittern handelt es sich um kleine Moleküle (Molekulargewicht weniger als 1 Kilodalton), die als chemische Kommunikationsmittel zwischen Neuronen wirken. Sie werden durch präsynaptische Neuronen synthetisiert und in den synaptischen Spalt freigesetzt, wo sie ihrerseits dann Rezeptoren an postsynaptischen Neuronen beeinflussen können (nach DE 69001672 T2).

Zu diesen neuroendokrinen Störungen / Erkrankungen gehören

- ADS / ADHS bei Kindern und Erwachsenen (Aufmerksamkeits-Defizit-Syndrom / Aufmerksamkeits-Defizit-Hyperaktivitäts-Syndrom - ADS mit Hyperaktivität). Dabei handelt es sich um eine komplexe Störung, die meist im sozialen Bezugssystem zu suchen ist. Als eine eventuelle medikamentöse Therapie wird die Behandlung mit Methylphenidat (Roche Lexikon Medizin, 5. Auflage, 2003) empfohlen (ferner: Attention-Deficit-Hyperactivity Disorder. MD Rappley; NEJM 2002; 352:165-73; Catecholamines in attention-deficit hyperactivity disorder: current perspectives. Pliszka SR, McCracken JT, Maas JW. J Am Acad Child Adolesc Psychiatry 1996 Mar; 35(3):264-72; The control of responsiveness in ADHD by catecholamines: evidence for dopaminergic, noradrenergic and interactive roles. Dev Sci 2005; 8: 122-31).

- CFS / das Chronisches Müdigkeitssyndrom - Chronic Fatigue Syndrom (Holmes GP, Kaplan JE, Gantz NM, Komaroff AL, Schonberger LB, Straus SE et al., Ann Intern Med. 1988, 108:387-9; Prins JB, van der Meer JWM, Bleijenberg G, Chronic Fatigue Syndrome, Lancet 2006; 367:346-355), s.a. Abschnitt [0008]. CFS ist ein klinisch definiertes Krankheitsbild, das durch schwere, lähmende Erschöpfung und eine Kombination von Symptomen charakterisiert wird, bei denen Konzentrationsschwäche und Merkfähigkeitsstörungen, Schlafstörungen, Muskel- und Gliederschmerzen im Vordergrund stehen (Fatigue in neurological disorders. Abhijt Chaudhuri, PO Behan. Lancet 2004; 363: 978-88; , Polymorphisms in genes regulating the HPA axis associated with empirically delineated classes of unexplained fatigue. Pharmacogenomics 2006; 7: 387-94; Combinations of single nucelotide polymorphisms in neuroendocrine effector and receptor genes predict chronic fatigue syndrome. Pharmacogenomics 2006; 7: 475-83).

**-** MCS / die Multiple Chemische Sensitivität (Ashford, N.A., Toxicology and Industrial Health, 1999, Nr. 3 S. 1-7 und dort zitierte Dokumente; Cullen, "Der Internist", Heft 1/98, S. 105 ff.). Bei Patienten mit dem Beschwerdebild einer MCS (multiplen Chemikalienüberempfindlichkeit, multiple chemical sensitivity) zeigt sich bei geringer Chemikalienexposition ein obligates systemisches Beschwerdebild. Die betroffenen Personen klagen über Gedächtnisstörungen, Schwäche, multiple Schmerzen im ganzen Körper, Schwindel, Kopfschmerzen, Schweißausbrüche, Atemnot, Zittern, Gelenk- und Muskelschmerzen o.ä.. Diese Reaktion wird durch verschiedenste Substanzen (z.B. Autoabgase, Parfüme, Lacke, Toner oder Holzschutzmittel) ausgelöst, unabhängig von deren chemischer Struktur und bei sehr niedrigen Konzentrationen, die normalerweise auf den Menschen keine subjektiv wahrnehmbaren Auswirkungen haben. Diese ungewöhnlichen Reaktionen wurden im Laufe der letzten 20 Jahre mit Begriffen wie Sick Building Syndrom (SBS), Chronisches Müdigkeitssyndrom (CFS), Environmental somatisation syndrome, Fibromyalgie, Building related illness o.ä. umschrieben. Weder sind bis heute die Ursachen dieses Krankheitsbildes eindeutig geklärt, noch gibt es klare diagnostische Parameter dafür. In der Regel lassen sich bei den betroffenen Personen keine erhöhten Chemikalienspiegel nachweisen. Es sind weder neurologische oder internistische Auffälligkeiten feststellbar, die klassischen Laborparameter sind ebenfalls normal. Ebenso wenig gibt es Anhaltspunkte dafür, dass allergische Reaktionen eine bedeutende Rolle spielen. Immer wieder diskutiert wurde u.a. eine Hyperreaktivität des Immunsystems als Ursache dieses Krankheitsbildes, wobei bisher keine experimentellen oder klinischen Anhaltspunkte für diese Hypothese aufgezeigt werden konnten. Bislang wurde keine somatische Ursache bzw. ein entsprechendes diagnostisches Kriterium für MCS gefunden, so dass vielfach dazu geneigt wird, MCS als psychosomatische Gesundheitsstörung einzustufen.

- FMS, die Fibromyalgie (Fibromyalgie-Syndrom, Jason LA et al., Psychosom Med 2000; 62:655-63), worunter Muskelfaserschmerzen fallen als eine chronische, nicht entzündliche Erkrankung ungeklärter Ursache, charakterisiert durch einen generalisierten Schmerz mit ausschließlichem Weichteilbefall ohne Gelenk- oder Knochenbefall, ohne laborchemische Veränderungen und ohne krankhaft veränderte bildgebende Diagnostik. Die Ursache der Fibromyalgie ist bislang ungeklärt. Verschiedene Theorien sind dazu entwickelt worden. Neben einer möglichen Virusinfektion wurden auch Autoimmunmechanismen sowie psychogene Ursachen (Roche Lexikon Medizin, 5. Auflage, 2003) vermutet (Hypothalamic-pituitarygonadal axis and cortisol in young women with fibromyalgia: the potential roles of depression, fatigue, and sleep disturbance in the occurrence of hypocortisolism, A Gur, R Cevik, AJ Sarac, L Colpan, S Em. Ann Rheum Dis 2004; 63: 1504-06; Neuroendocrine and immune aspects of fibromyalgia, D van West, M Maes; BioDrugs 2001; 15: 521-31. Fibromyalgia and the serotonin pathway, JH Juhl, Altern Med Rev 1998, 3: 367-75; Cytokines playan aetiopathogenetic role in fibromyalgia: a hypothesis and pilot study. Rheumatology 2001, 40: 743-49).

- Kopfschmerzen / Migräne, worunter anfallsweise, funktionelle, sich periodisch wiederholende, meist halbseitige (Hemicrania) Kopfschmerzen mit neurologischen Störungen (Par- u. Anästhesien, Paresen, aphasische Störungen) fallen, die die eigentliche Kopfschmerz-Symptomatik überdauern. Zur Vorbeugung oder Behandlung werden Migränemittel empfohlen, wie Analgetica (z.B. ASS, Ibuprofen, Paracetamol) in Kombination mit Prokinetika, Triptane (5-HT1-Agonisten) oder Mutterkornalkaloide (z.B. Dihydroergotamin, Ergotamintartrat) und zur Migräne-Prophylaxe vor allem Betarezeptorenblocker (Metoprolol, Propranolol), alternativ Flunarizin als Calciumantagonist (Roche Lexikon Medizin, 5. Auflage, 2003), des weiteren Antidepressiva, Serotoninantagonisten oder Entzündungshemmer (Migraine - current understanding and treatment. PJ Goadsby, RB Lipton, MD Ferrari. NEJM 2002; 346: 257-70; Migraine, SD Silberstein. Lancet 2004; 363: 381-91; Preventive treatment of migraine, SD Silberstein 2005; Rev Neurol Dis 2: 167-75; Plasma 5-HT in migraine during an attack-free period. Headache 2006; 46: 592-96).

- Übergewicht / Adipositas (Obesitas; Fettleibigkeit, worunter meist generalisierte Vermehrung des Fettgewebes und übermäßige Körpergewichtserhöhung infolge positiver Energiebilanz (Roche Lexikon Medizin, 5. Auflage, 2003) verstanden wird (Urinary epinephrine and norepinephrine interrelations with obesity, insulin, and the metabolic syndrome in Hong Kong Chinese Lee ZS, Critchley JA, Tomlinson B, Young RP, Thomas GN, Cockram CS, Chan TY, Chan JC.Metabolism 2001; 50:135-43; , Masaki T, Chiba S, Yasuda T, Noguchi H, Kakuma T, Watanabe T, Sakata T, Yoshimatsu H. Involvement of hypothalamic histamine H1 receptor in the regulation of feeding rhythm and obesity. Diabetes. 2004 Sep; 53(9):2250-60; Seeley RJ, York DA. Fuel sensing and the central nervous system (CNS): implications for the regulation of energy balance and the treatment for obesity. Obes Rev. 2005 Aug;6(3):259-65. Review; Stanley S, Wynne K, McGowan B, Bloom S. Hormonal regulation of food intake. Physiol Rev. 2005 Oct;85(4):1131-58; Obesity and Cortisol. P Björntorp, R Rosmond. Nutrition 2000; 16: 934-36). In der Patentschrift EP 1593312 werden Nahrungsmittel oder Nahrungsergänzungsmittel gegen Adiposität und erhöhte Blutlipidwerte beschrieben, deren Wirksamkeit darauf beruht, dass sie Lysin enthalten.

- Kohlehydrat-Heißhunger (Craving): zu den Essstörungen gehörende Sucht, große Nahrungsmengen unkontrolliert zu verschlingen mit anschließendem Erbrechen (Bulimie), oft einhergehend mit depressiven Verstimmungen (Roche Lexikon Medizin, 5. Auflage, 2003) nach der Nahrungsaufnahme (Hypothalamic dopamine and serotonin in the regulation of food intake. Meguid MM, Fetissov SO, Varma M, Sato T, Zhang L, Laviano A, Rossi-Fanelli F. Nutrition 2000t;16:843-57; Diet, monoamine neurotransmitters and appetite control Fernstrom JD, Fernstrom MH. Nestle Nutr Workshop Ser Clin Perform Programme 2001; 5: 117-31; Central nervous system biogenic amine targets for control of appetite and energy expenditure. DL Nelson, DR Gehlert. Endocrine. 2006 Feb; 29(1):49-60. Review).

- Appetitstörungen: Sie sind heute ein weit verbreitetes Phänomen (The role of stress and the HPA axis in the pathogenesis of the metabolic syndrome: neuroendocrine and target tissue-related causes. GP Chrousos. It J Obesity, 2000; 24: S.50-55). Für die Verdauungsschwäche sind in den meisten Fällen mehrere Ursachen verantwortlich. Besonders schwerwiegend wirkt sich eine mangelhafte Gallensekretion aus. Ohne therapeutischen Eingriff kann sich ein Teufelskreis entwickeln, der zu einer dauerhaften Beeinträchtigung des gesamten Organismus führt (www.sidroga.com/16.html).

- Depressionen (Larson et al., 2001, Brain Behaviour Immunity, 15:371-387), primäre und sekundäre (Stress and the brain: from adaptation to disease. E R de Kloet, M Joels, F Holsboer. Nature Rev 2005; 6: 463-75; The potential role of hypocortisolism in the pathophysiology of stress-related bodily disorders. C Heim, U Ehlert, DH Hellhammer. Psychneuroendocrinol 2000; 25: 1-35; Crossroads of corticotropin releasing hormone, corticosteroids and monoamines. About a biological interface between stress and depression. Neurotoxicity Res 2002; 4: 531-555).

- Schlafstörungen (Holmes GP, Kaplan JE, Gantz NM, Komaroff AL, Schonberger LB, Straus SE et al., Ann Intern Med. 1988; 108:387-9; Prins JB, van der Meer JWM, Bleijenberg G, Lancet 2006; 367:346-355).

- Angstsyndrome /Panikattacken (von gr. Pan Παv, dem Hirtengott und Dämon des Schreckens), wozu Zwangshandlungen, soziale Phobien und andere Formen der Angststörung gehören, auch Klaustrophobie (krankhafte Furcht vor geschlossenen Räumen). Angst stellt einen als unangenehm empfundenen, eine Bedrohung oder Gefahr signalisierenden emotionalen Gefühlszustand dar, der dann einen Krankheitswert erhält, wenn er ohne erkennbaren Grund bzw. infolge inadäquater Reize ausgelöst und empfunden wird (a. Angststörung, Angstneurose, Angst-Glück-Psychose). Panikattacken sind plötzlich einsetzende, zeitlich umschriebene, von Angst oder intensiver Besorgnis geprägte Perioden, häufig verbunden mit Atemnot, Vernichtungsgefühl, dem Gefühl eines drohenden Unheils und der Befürchtung, den Verstand zu verlieren (Pschyrembel Klinisches Wörterbuch, 260. Auflage 2004). Panikattacken, Zwangshandlungen, soziale Phobien und andere Formen der Angststörung gehören zu den häufigsten psychischen Erkrankungen: Ein bis zwei Prozent der Bevölkerung sind in Deutschland betroffen. Obwohl sich die medizinische Versorgung der Patienten in den letzten Jahren verbessert hat, ist diese nach wie vor unbefriedigend (Panic Disorder. WJ Keaton. NEJM 2006; 354: 2360-67).

- Nahrungsmittelunverträglichkeit (food intolerance) stellt eine Sammelbezeichnung (Pschyrembel Klinisches Wörterbuch, 260. Auflage 2004) für Erkrankungen dar, die entweder allergisch bedingt sind oder auf einem Verdauungsenzym-Mangel beruhen und die zu klinischen Symptomen wie Hautausschlag, Erbrechen oder Durchfall führen (Chua AS, Keeling PW.Cholecystokinin hyperresponsiveness in functional dyspepsia. World J Gastroenterol. 2006;12:2688-93; Central serotonergic and noradrenergic receptors in functional dyspepsia. O'Mahony S, Dinan TG, Keeling PW, Chua AS. World J Gastroenterol. 2006 May 7;12(17):2681-7. Review).

**-** Prämenstruelles Syndrom (PMS) bzw. Prä- /Perimenopause-Beschwerden (Premenstrual syndrome. K Wyatt. Clin Evid. 2003 Jun;(9):2125-44. Neuroendocrine effects on mood. MG spinelli. Rev Endocr Metab Disord. 2005 May;6(2):109-15; Pre-menstrual syndrome: a new concept in its pathogenesis and treatment. AM Levin. Med Hypotheses. 2004;62(1):130-2; The etiology, biology, and evolving pathology of premenstrual syndromes. U Halbreich. Psychoneuroendocrinology. 2003 Aug;28 Suppl 3:55-99). Hierbei handelt es sich um körperliche und psychische Beschwerden, die in der 2. Hälfte des Regelzyklus (Gelbkörperphase) immer wiederkehren. Es wird angenommen, dass etwa 20 % aller Frauen prämenstruelle Beschwerden haben, die jedoch nicht immer behandlungsbedürftig sind. Zu den körperlichen Beschwerden, wie Brustbeschwerden (Mastodynie), Völlegefühl, Blähungen, Kopfschmerzen, Konzentrationsschwäche, zyklische Gewichtszunahme ("Ess-attacken"), Wassereinlagerung (Ödembildung), Schwächegefühl bis zur Erschöpfung, Akne, Unterleibsschmerzen, gesellen sich auch neuro-vegetative Beschwerden, wie Reizbarkeit, schwankende Gemütszustände, depressive Verstimmung (PD Dr. med. Ronald Warm, Facharzt für Gynäkologie; Copyright © 2006 Qualimedic AG;
frauen.qualimedic.de/Praemenstruelles_syndrom.html).

**-** Irritables Kolon (Reizdarm, Reizkolon): Funktionelle Darmstörung ohne nachweisbare biochemische oder strukturelle Normabweichung (Pschyrembel Klinisches Wörterbuch, 260. Auflage 2004), die häufig kombiniert mit anderen Beschwerden (Migräne, Menstruationsbeschwerden, Palpitationen) auftritt (s.a. Hypothalamic-pituitary-gut axis dysregulation in irritable bowel syndrome: plasma cytokines as a potential biomarker? Dinan TG, Quigley EM, Ahmed SM, Scully P, O'Brien S, O'Mahony L, O'Mahony S, Shanahan F, Keeling PW. Gastroenterology. 2006;130:304-11; Review article: intestinal serotonin signalling in irritable bowel syndrome. GM Mawe, GM Coates, PL Moses. Aliment Pharmacol Ther. 2006 Apr 15;23(8):1067-76. Review).

- Burn-Out-Syndrom (Larson et al. 2001, Brain Behaviour Immunity, 15:371-387; ferner: Burnout, percweived stress, and cortisol responses to awakening. JG Pruessner, DH Hellhammer, C Kirschbaum. Psychosom Med 1999; 61: 197-204).

- Sickness-Syndrom (Larson et al. 2001, Brain Behaviour Immunity, 15:371-387).

- Muskel- und Gliederschmerzen (Holmes GP, Kaplan JE, Gantz NM, Komaroff AL, Schonberger LB, Straus SE et al., Ann Intern Med. 1988; 108:387-9; Prins JB, van der Meer JWM, Bleijenberg G, Lancet 2006; 367:346-355)

- Konzentrations- und Merkfähigkeitsstörungen, Gedächtnisschwäche (Kurzzeitgedächtnis): Prins JB, van der Meer JWM, Bleijenberg G, Lancet 2006; 367:346-355; Holmes GP, Kaplan JE, Gantz NM, Komaroff AL, Schonberger LB, Straus SE et al., Ann Intern Med. 1988; 108:387-9).

- Koordinationsstörungen: Unter diesem Begriff fasst man alle möglichen körperlichen Störungen zusammen, die sich auf das richtige Koordinieren von Bewegungsabläufen beziehen. Die essenziellen Koordinationsvorgänge werden in der Kindheit erlernt, z.B. Gehen-Lemen, Hände einsetzen. Dagegen werden nichtessenzielle Koordinationsvorgänge oft durch Gewohnheit oder Training erlernt. Eine weitere mögliche Ursache für Störungen stellen neurologische Schäden dar. Sie betreffen das Gehirn und das Nervensystem. Eine Unterbrechung der Nervenbahnen kann auch durch bestimmte Medikamente und Drogen bewirkt werden (toxikologische Ursachen). Die Inhaltsstoffe blockieren hierbei insbesondere die Schaltstellen zwischen den Nerven, die sogenannten Neurotransmitter, sodass das Signal nicht bzw. nur teilweise weitergeleitet wird (ein Beispiel ist der "Tatterich" bei Kaffeetrinkern oder Rauchern). Manche Koordinationsstörungen können auch vererbt werden. Bei den "angelernten" Koordinationsstörungen können durch die fehlende Übung ruckartige Bewegungen auftreten, auch Krämpfe und Verspannungen sind möglich. Tremor ist eine häufige Bewegungsstörung, die sich in unwillkürlichem rhythmischen Zittern äußert (de.wikipedia.org/wiki/Koordinationsstörungen). Gestörte Koordination bewirkt z.B. ruckartige bzw. fahrige Bewegungen, Krampf, Verspannungen oder Schielen (de.wikipedia.org/wiki/Koordination).

- CMI (Chronic Multisystem Illnesses; Jason LA et al., Psychosom Med 2000; 62:655-63).

- Sick-Building Syndrome (SBS, Building related illness, Gebäudeabhängige Erkrankungen, Environmental somatisation syndrome, Idiopatic Environmental Intolerance; Larson et al. 2001, Brain Behaviour Immunity, 15:371-387). SBS wird auf der Internetseite (http://www.medizinfo.com/umweltmedizin/syndrome/sickbuilding.htm) folgendermaßen charakterisiert: Symptome und Verlauf sind Kopfschmerzen, Müdigkeit, Konzentrationsschwäche, Depression, Vergesslichkeit, Empfindungsstörungen an Händen, Füßen, Armen und Beinen, unklare Schmerzen, Reizerscheinungen an Augen, Atemwegen und Haut. Die Symptome klingen im Freien ab und treten in geschlossenen Räumen erneut auf. Ursachen können überheizte Räume, niedrige Luftwechselraten in geschlossenen Räumen, Schimmelpilzsporen, chemische Ausdünstungen, oft auch Klimaanlagen, welche die Luft nur umwälzen und nicht erneuern, sein. SBS gehört zu jenen Reaktionen normaler Versuchspersonen auf bekannte oder unbekannte Expositionen, die sich mit klassischen oder bekannten Mechanismen nicht erklären lassen, deren Symptome aber verschwinden, wenn sie der vom Gebäude ausgehenden Belastung nicht mehr ausgesetzt sind.
Oft wird MCS und das SBS fälschlicherweise als das Gleiche angesehen. MCS unterscheidet sich jedoch vom Sick Building Syndrome durch das Auftreten von Symptomen in jeder Umgebung, innen oder außen, in der eine betroffene Person gegenüber einer Chemikalie exponiert ist. Gebäudeabhängige Erkrankungen kommen nur in speziellen Innenräumen, wie z.B. Bürogebäuden, vor und können zusätzlich auch durch Inhalationsallergene, wie Schimmelpilze, unter Umständen gepaart mit Stress am Arbeitsplatz oder mit Temperaturschwankungen, also nicht nur durch Chemikalienexposition, ausgelöst werden (Kreiss, The epidemiology of building-related complaints and illness, Occup. Med: State of the Art Reviews.1989).

- Übertrainingssyndrom, OTS: Overtrained Athlete Syndrome (Hormonal responses in athletes: the use of a two bout exercise protocol to detect subtle differences in (o-ver)training status. R Meeusen et al. Eur J Appl Physiol. 2004; 91:140-6. oder The overtraining syndrome in athletes: a stress-related disorder. A Angeli, M Minetto, A Dovio, P Pancotti. J Endocrinol Invest. 2004;27:603-12; Chronic fatigue syndrome. A brief review of functional disturbances and potential therapy. RJ Shephard. J Sports Med Phys Fitness. 2005;45:381-92). Das Übertrainings-Syndrom stellt ein Phänomen vorwiegend des Ausdauersports oder des Kraftsport dar und kommt auch in anderen Stresszuständen vor, wie z.B. bei beruflicher Überlastung. In der Wissenschaft spricht man vom OTS (overtraining syndrome) und zunehmend vom UPS (underperformance syndrome). Ursache des Übertrainingssyndroms bzw. - zustandes ist eine für den Trainingszustand zu hohe Trainingsintensität, sodass eine ausreichende Regeneration zwischen den Trainingseinheiten nicht mehr gewährleistet ist und es zunächst zu einer Leistungsstagnation und schließlich zum Leistungsabfall kommt (gin.uibk.ac.at/thema/ sportundemaehrung/uebertraining.html;
gin.uibk.ac.at/thema/sportundernaehrung/ Das_Uebertrainingssyndrom.pdf).

- SAD / Saisonal Affective Disorder (Seasonal Affective Disorder and HPA Axis Disturbance. Schuppenies A, P Schwarz, SR Bornstein. Horm Metab Res. 2006 Jun;38(6):434. The circadian basis of winter depression. AJ Lewy, BJ Lefler, JS Emens, VK Bauer. Proc Natl Acad Sci U S A. 2006;103:7414-9). Unter "Saisonal Abhängige Depression" (SAD) wird eine Unterform der depressiven Erkrankung verstanden, die regelmäßig zu einem bestimmten Zeitpunkt im Jahr auftritt (meist im Herbst). Nur etwa ein Prozent der Allgemeinbevölkerung ist davon betroffen. Im Vordergrund dieser Depressionsform steht das Erleben mangelnder Energie und verminderten Antriebs, aber auch viele andere Symptome, die sonst üblicherweise bei depressiven Erkrankungen auftreten, wie eine niedergedrückte Stimmung, Schuldgefühle und Freudlosigkeit (www.medizinauskunft.de).

- Stress, chronischer Stress sowie das Posttraumatische Stresssyndrom PTSD: Post traumatic Stress Disorder (Stress and the brain: from adaptation to disease. E R de Kloet, M Joels, F Holsboer. Nature Rev 2005; 6: 463-75; The potential role of hypocortisolism in the pathophysiology of stress-related bodily disorders. C Heim, U Ehlert, DH Hellhammer. Psychneuroendocrinol 2000; 25: 1-35; Endocrinology of the stress response. E Charmandari, C Tsigos, G Chrousos. Annu Rev Physiol 2005; 67: 259-84). Zu den wichtigsten Mediatoren der pathologischen Stress-Antwort wird das inflammatorische Zytokin IL1 gerechnet (Konsman et al, Trends Neurosci 25: 154-159, 2002). Es besteht demnach ein neuroendokrinimmunologischer Regelkreis: Nervenzellen weisen Rezeptoren für Zytokine wie IL-1 oder TNF-alpha auf, und umgekehrt verfügen Immunzellen über Rezeptoren für biogene Amine wie Adrenalin, Noradrenalin, Dopamin oder Serotonin (Haas, Schauenstein, Allergy 56:470-477, 2001). So stimuliert IL1 die Freisetzung von Cortisol, IFN-gamma hemmt die Serotoninsynthese über die Aktivierung des Enzyms IDO (Indolamin-2,3-Dioxygenase) mit verstärktem Abbau von Tryptophan zu Kynurenin (Munn et al, J Exp Med, 189: 1363-1372, 1999). Unter chronischem Stress nimmt die Ausschüttung von biogenen Aminen ab, auch durch die eingeschränkte Verfügbarkeit von Vorläufermolekülen wie Tyrosin (4-Hydroxy-phenylalanin) oder Tryptophan (Indolyl-3-alanin). Erkrankungen mit pathologischer Stress-Response wie das Burn-Out-Syndrom oder die sog. Sickness-Behaviour gehen deshalb mit einer inflammatorischen Dysregulation der Immunabwehr einher (Larson et al. 2001, Brain Behaviour Immunity,15:371-387).

In zahlreichen Publikationen sind wiederholt Zusammenhänge zwischen Fatigue, CFS, FMS, MCS (Abkürzungsverzeichnis hinter den Beispielen) und anderen umweltassoziierten Gesundheitsstörungen sowie pathologischen Veränderungen der Neurostresshormone CRH (Corticotropin-Relasing Hormon), Cortisol, DHEAS, Prolactin, Wachstumshormon, Melatonin und der Neurotransmitter Katecholamine, Serotonin, PEA beschrieben worden (Friedman EM, Lawrence DA, Environmental stress mediates changes in neuroimmunological interactions, Toxicol Sci 2002, 67:4-10; Fatigue in neurological disorders, Abhijt Chaudhuri, PO Behan Lancet 2004, 363: 978-88).

Das Posttraumatische Stresssyndrom (PTSD) steht im Zusammenhang mit der Verarbeitung eines Traumas nach Katastrophen (Posttraumatische Belastungsstörung). Nach Angaben der Deutschsprachigen Gesellschaft für Psychotraumatologie (DeGPT) leiden bis zu 12 Millionen Menschen im deutschsprachigen Raum an einer Posttraumatischen Belastungsstörung, die durch Extrem-Stress-Ereignisse verursacht werden (www.medizin.de/gesundheit; (www.medizin.de/gesundheit/deutsch/863.htm).

Forscher weltweit haben herausgefunden, dass bis zu 75% der Bevölkerung im Laufe ihres Lebens eine traumatische Erfahrung machen. 25% davon entwickeln eine Trauma-Folgeerkrankung. Verkehrsunfälle, der Verlust einer nahen Bezugsperson, Kriege oder Gewalt sind häufig traumatisierende Erlebnisse, auf die Menschen mit Angst, Hilflosigkeit und Entsetzen reagieren. Die Verarbeitung braucht viel Zeit, und nur ein Drittel der Betroffenen ist in der Lage, das Trauma aus eigener Kraft zu überwinden (www.medizin.de/gesundheit/deutsch/1336.htm).

- Der Zusammenhang zwischen Stress, Depression, Motivation, Kognition, Aufmerksamkeit, Schlaf, Ängsten, Appetitverhalten, Energiestoffwechsel, Schmerzempfindungen einerseits und neuroendokrinen Regelmechanismen andererseits ist Gegenstand intensiver Forschung. Hypercortisolismus gilt als das zentrale biochemische Kriterium der primären Depression, Burn-Out als pathologische Spätmanifestation des chronischen Stresses (Gold PW, Chrousos GP; Organization of the stress system and its dysregulation in melancholic and atypical depression: high versus low CRH/NE states, Mol Psychiatry 2002; 7: 254-75; de Kloet ER, Joels M, Holsboer F; Stress and the brain: from adaptation to disease. Nat Rev 2005; 6:463-75; Charmandari E, Tsigos C, Chrousos G: Endocrinology of the stress response, Annu Rev Physiol 2005;67:259-84).

In der Patentliteratur ist Behandlung von Neurotransmitterstörungen mehrfach bechrieben worden:

Um die Behandlung derartiger Störungen geht es z.B. in der DE-OS (deutsche Offenlegungsschrift Nr.) 10260263 A1 (von 2004), darüber hinaus auch um die Ernährung von Patienten, die unter Aminosäurestoffwechselstörungen leiden. In dieser Schrift werden Tetrahydrobiopterin oder Derivate davon als die wirksamen Verbindungen unter Schutz gestellt, die einem erhöhten Gehalt an Phenylalanin und einem verminderten Gehalt an Tyrosin, Serotonin oder Dopamin im Körper entgegenwirken sollen.

In der DE 10352129 A1 geht es um den Einsatz von Organozinkverbindungen für die Nahrungsergänzung. Die aus Zinkoxid und Aminosäuren bzw. Aminosäuregemischen erhaltenen Organo-Zinkverbindung sind als Nahrungsergänzungsmittel vorgesehen, die ggf. einen Anteil an für die Reaktion im Überschuss zugegebenes Zinkoxid enthalten können.

Der DE 10238162A1 liegt ein Nahrungsergänzungsmittel aus Nähr- und Vitalstoffen zugrunde, das Mineralstoffe, Spurenelemente, Vitamine, Aminosäuren, Peptide, Coenzym Q 10, Ethylaminophosphat oder sekundäre Pflanzenstoffe auf lebensmittelrechtlich zugelassenen Trägermitteln aus Polymeren, Cellulosen, Verdickungsmitteln oder etwa Tensiden enthält.

In der DE 10206159 A1 werden Mittel beschrieben, die Folsäure, Vitamin B6 und Vitamin B12 enthalten und die vor allem zur vorbeugenden und akuten Behandlung von vaskulären und neurodegenerativen Erkrankungen eingesetzt werden sollen.

Um die Verwendung von Phosphatidylserin zur Behandlung des Aufmerksamkeits-Defizit-Syndroms (ADHS) geht es in der DE 10149108 A1.

Die EP 1593312 beschreibt Nahrungsergänzungsmittel gegen Adiposität und gegen erhöhte Blutlipidwerte, die als wirksamen Bestandteil Lysin enthalten.

In weiteren Nahrungsergänzungsmitteln, auch solchen für Mensch und Tier, sind als wirksame Bestandteile Vitamin E (DE 19805161 A1) oder Patothensäure (DE 9412374 U1) vorgeschlagen worden, in anderen filmbildende Überzugsmittel (DE 10260919A1) für Nahrungsergänzungsmittel mit Langzeitwirkung (DE 10103480A1).

Mit der Europäischen Patentschrift EP 0478671 B1 (WO 90/15637; DE 69001672 T2 von 1993) wird ein neurologisches Therapiesystem unter Schutz gestellt, das eine Behandlung von Neurotransmitter-Defizienz- und Fehlfunktions-Erkrankungen durch lokale und kontrollierte Abgabe eines Neurotransmitters an das Gehirn zum Inhalt hat.

Gemäß der EP 0554352 B1 (WO 92/006702; DE 69133205 T2 von 2003) wird zur Behandlung von neurologischen Störungen vorgeschlagen, eine Implantation oder Transplantation von lebensfähigen Zellen in das Gehirn vorzunehmen.

Zur Behandlung neurodegenerativer Erkrankungen sind als Wirkstoffe auch polycyclische Heteroaryl-Verbindungen vorgeschlagen worden (z.B. gegen AD: Alzheimer's Desease), wie Thiazolyle oder Benzothiazolyle (CA 02488979 A1), auch hydrogenierte Pyrido(4,3-b)indole (gegen Parkinsonsche Krankheit, Sklerosen), Tacrine oder z.B. 9-Amino-1,2,3,4-tetrahydroacridin-Hydrochlorid (EP 0876818 B1) sowie Benzamide (EP 0819113 B1) oder Arylcyclohexylamine zum Neuroschutz (EP 0396734 B1).

Die Nachteile der beschriebenen Lösungen zur Behandlung der Erkrankungen liegen vor allem darin, dass es bisher schwer ist zu erkennen, welche Störung denn nun wirklich vorliegt und welche bekämpft werden soll. Im Wesen einer Ausschlussdiagnostik liegt es, dass sie zwar andere Krankheiten ausschließen kann, aber keinen gesicherten Hinweis auf MCS, CFS, SBS o.a. gesundheitliche Störungen liefert. Auch wurde bisher angenommen, dass beispielsweise aufgrund der Unterschiede zwischen MCS und SBS eine einheitliche Diagnose und eine gezielte Behandlung überhaupt nicht möglich ist.

Der Erfindung liegt die Aufgabe zugrunde, die Nachteile der bekannten Lösungen zu beseitigen und neue Möglichkeiten zur Behandlung von neuroendokrinen gesundheitlichen Störungen, insbesondere von Neurotransmitter- /Stresshormon-Ungleichgewichten des neuroendokrinen Regelkreises, zu eröffnen.

Die Aufgabe wurde durch die Verwendung von Neurotransmitter-Derivaten zur Herstellung von Mitteln gelöst, die zur Behandlung von neuroendokrinen gesundheitlichen Störungen geeignet sind. Derartige Störungen gehen mit Stresshormon- und /oder Neurotransmitter-Ungleichgewichten des neuroendokrinen Regelkreises einher. Den erfindungsgemäßen Mitteln liegen als wirksame Komponenten Neurotransmitter-Derivate - Aminosäurevorstufen von Neurotransmittern, Kofaktoren für die Synthese von Neurotransmittern und Neurotransmitter-Modulatoren - zugrunde.

Die den erfindungsgemäßen Mitteln zugrundeliegenden Aminosäurevorstufen bestehen aus Tyrosin und /oder Acetyltyrosin und /oder Phenylalanin und /oder 5-Hydroxytryptophan (5-HTP) und /oder Mucuna und /oder Glutamin und /oder aus Histidin, die Neurotransmitter-Modulatoren aus Taurin und /oder Theanin und /oder Rhodiola rosea und /oder Grüntee (EGCG) und /oder aus Phosphatidylcholin und /oder aus Phosphatidylserin und die Kofaktoren für die Synthese von Neurotransmittern aus den Vitaminen C und /oder B3, B5, B6, B 12 und /oder Vitamin E (Tocopherole) und /oder N-Acetylcystein und /oder Folat und /oder aus Magnesium-, Calcium-, Selen-, Zink-, Mangan- und /oder aus Chrom-Verbindungen.

Die erfindungsgemäßen Mittel sind zur Behandlung von neuroendokrinen gesundheitlichen Störungen, Neurostresszuständen, Neuroregulationsstörungen oder Regulationsstörungen der neuroendokrinen Stressachse der Art
a) ADS /ADHS (bei Kindern, Erwachsenen)
b) CFS /Chronisches Müdigkeitssyndrom
c) Fatigue (Müdigkeit /Erschöpfbarkeit)
d) MCS /Multiple Chemische Sensitivität
e) Fibromyalgie
f) Kopfschmerzen /Migräne
g) Übergewicht /Adipositas
h) Kohlehydrat-Heißhunger
i) Appetitstörungen
j) Depressionen
   j1) primäre, endogene Depressionen / rezidivierende depressive Episoden
   j2) sekundäre, reaktive oder somatogene Depressionen / depressive Episoden
   j3) Stress-Depressionen
   j4) Winter- /Frühjahrsdepressionen
   j5) Depressionen infolge Pharmakotherapie (Zytokine, Zytostatika)
k) Angstsyndromen
   k1) Panikattacken
   k2) Klaustrophobie
l) Schlafstörungen
m) Nahrungsmittelunverträglichkeiten
n) Prämenstruelles Syndrom
o) Prä- /Perimenopause-Beschwerden
p) Irritables Kolon (Reizdarm)
q) Burn-Out-Syndrom
r) Sickness-Syndrom
s) Muskelschmerzen
t) Konzentrationsstörungen
u) Gedächtnisschwäche (Kurzzeitgedächtnis)
v) Koordinationsstörungen
w) CMI (Chronic Multisystem Illnesses)
x) Sick-Building Syndrome (SBS)
y) Building related illness
z) Environmental somatisation syndrome
α) Idiopatic Environmental Intolerance
β) Übertrainingssyndrom (OTS: Overtrained Athlete Syndrome)
γ) SAD / Saisonal Affective Disorder
δ) Übertrainingssyndrom (OTS: Overtrained Athlete Syndrome)
ε) Saisonal Affective Disorder, Winterdepression
ζ) Chronischer Stress
η) Posttraumatisches Stresssyndrom (PTSD: Post traumatic Stress Disorder)
ϑ) Autismus
geeignet.

Die erfindungsgemäßen Mittel stellen auch Nahrungsergänzungsmittel (NEM) dar.

Als NEM1 bis NEM8 enthalten sie gemeinsam Aminosäurevorstufen von Neurotransmittern, Neurotransmitter-Modulatoren, Kofaktoren für die Synthese von Neurotransmittern, Vitamin C und Vitamine der Reihe B.

Das Nahrungsergänzungsmittel NEM1 enthält L-Glutamin, L-Tyrosin und L-Histidin als wesentliche Komponenten. Als NEM1A besteht es aus zusätzlich Vitamin B5 und /oder Vitamin C, Vitamin B6, Vitamin B3, Vitamin B12, Folsäure, Magnesium, Zink, Rhodiola rosea und /oder Crrün-Tee-Extrakt. Bei Rhodiola rosea (Rosenwurz) handelt es sich um ein Naturprodukt [Bioactive compounds from Rhodiola rosea (Crassulaceae), DS Ming et al. Phytother Res. 2005 Sep;19:740-3]. Grün-Tee-Extrakt (EGCG: Epigallocatechin-3-gallat) stellt ist ein natürliches Produkt aus dem grünen Tee dar (Epigallocatechin-3-gallate, DG Nagle, D Ferreira, YD Zhou. Phytochemistry. 2006 Jul 28).

Das Nahrungsergänzungsmittel NEM2 besteht in seinen wesentlichen Komponenten aus Theanin, 5-Hydroxytryptophan (5-HTP) und Mucuna pruriens. Theanin ist eine Glutaminähnliche Aminosäure (Gamma-Glutaminyl-Ethylamid), die fast ausschließlich in der Teepflanze vorkommt (Sakato, J.: Agr.Chem.Soc.Japan 23 (1950) 262-267). Mucuna pruriens stellt eine natürliche Quelle von 3,4-Dihydroxyphenylalanin dar (L-Dopa), ein Zwischenprodukt in der Dopaminsynthese [Distribution of L-DOPA in the root of velvet bean plant (Mucuna pruriens L.) and gravity. K Tomita-Yokotani et al. Biol Sci Space. 2004; 18:165-6]. Als Nahrungsergänzungsmittel besteht NEM2A aus zusätzlich Vitamin B6 und /oder Folsäure, Vitamin B12, Magnesium, Rhodiola rosea, Taurin (1-Amino-ethan-2-sulfonsäure) und /oder Phosphatidylcholin (Lecithin) bzw. Phosphatidylserin ("Brain-specific" nutrients: a memory cure? MA McDaniel, SF Maier, GO Einstein. Nutrition. 2003 Nov-Dec;19(11-12):957-75. Review).

Das Nahrungsergänzungsmittel NEM3 besteht in seinen wesentlichen Komponenten aus L-Tyrosin (β-(4-Hydroxy-phenyl)-alanin) und Mucuna pruriens. Als NEM3A besteht es aus zusätzlich Vitamin C und /oder Vitamin B6, Folsäure, Calcium, Selen und /oder N-Acetyl-Cystein.

Das Nahrungsergänzungsmittel NEM4 besteht in seinen wesentlichen Komponenten aus L-Tyrosin und L-Histidin. Als NEM4A besteht es aus zusätzlich Vitamin C und /oder Vitamin B5, Vitamin B6, Vitamin B3, Folsäure, Magnesium, Zink, Mangan, N-Acetyl-Cystein und /oder Rhodiola rosea.

Das Nahrungsergänzungsmittel NEM5 besteht in seinen wesentlichen Komponenten aus L-Glutamin, Theanin, 5-HTP und L-Tyrosin. Als NEM5A besteht es aus zusätzlich Vitamin C und /oder Vitamin B6, Folsäure, Magnesium, Taurin, und /oder Tocopherolen /Tocotrienolen.

Das Nahrungsergänzungsmittel NEM6 besteht in seinen wesentlichen Komponenten aus Mucuna pruriens und 5-HTP. Als NEM6A besteht es aus zusätzlich Vitamin B3 und /oder Vitamin B5, Vitamin B6, Folsäure, Vitamin B12, Zink, Chrom, Selen, Mangan, N-Acetyl-Cystein und /oder Grün-Tee-Extrakt.

Das Nahrungsergänzungsmittel NEM7 besteht in seinen wesentlichen Komponenten aus L-Tyrosin, 5-HTP und Theanin. Als NEM7A besteht es aus zusätzlich Vitamin C und /oder Vitamin B6, Folsäure, Calcium und /oder L-Phenylalanin.

Das Nahrungsergänzungsmittel NEM8 besteht in seinen wesentlichen Komponenten aus 5-HTP und Theanin. Als NEM8A besteht es aus zusätzlich Vitamin C und /oder Vitamin B6, Folsäure, Vitamin B 12, Magnesium, Zink, Selen, Taurin und /oder N-Acetyl-Cystein.

Überraschenderweise hatte sich herausgestellt, dass durch den Nachweis von Neurosteroiden und von Neurotransmittern bzw. ihren Metaboliten in Körperflüssigkeiten Rückschlüsse auf neuroendokrine Störungen des neurovegetativen Regulationsgleichgewichts gezogen werden können und dadurch Behandlungen der damit verbundenen Erkrankungen mit Hilfe von Aminosäurevorstufen von Neurotransmittern, Neurotransmitter-Modulatoren und Kofaktoren für die Synthese von Neurotransmittern möglich sind.

Die erfindungsgemäßen Mittel können in ihren basischen Bestandteilen auch in Form ihrer physiologisch verträglichen Salze vorliegen. Dazu gehören die durch Neutralisation mit geeigneten anorganischen oder organischen Säuren hergestellten Salze, z.B. mit Salzsäure, Schwefel- oder Phosphorsäure, Ameisen-, Essig-, Propion-, Glykol-, Milch-, Brenztrauben-, Malein-, Furmar-, Anthranyl-, Naphthalinsulfon-, Sulfanyl- oder Zimtsäure.

Die Merkmale der Erfindung gehen aus den Elementen der Ansprüche und aus der Beschreibung hervor, wobei sowohl einzelne Merkmale als auch mehrere in Form von Kombinationen vorteilhafte Ausführungen darstellen, für die mit dieser Schrift Schutz beantragt wird.

Das Wesen der Erfindung besteht in einer Kombination aus bekannten (den Aminosäuren und ihren Derivaten) und neuen Elementen (ihrer Verwendung zu einem neuen Zweck), die sich gegenseitig beeinflussen und in ihrer neuen Gesamtwirkung einen Gebrauchsvorteil und den erstrebten Erfolg ergeben, der darin liegt, dass nunmehr Mittel zur Verfügung stehen, die eine Behandlung von Neuroregulationsstörungen aufgrund von Stresshormon- und /oder Neurotransmitter-Defiziten, -Überschüssen bzw. -Störungen des neuroendokrinen Regulationsgleichgewichts ermöglichen.

Die erfindungsgemäße Verwendung von Aminosäurevorstufen von Neurotransmittern, von Kofaktoren für die Synthese von Neurotransmittern und von Neurotransmitter-Modulatoren liegt darin, dass sie zur Herstellung von Mitteln zur Behandlung von neuroendokrinen gesundheitlichen Störungen, die mit Stresshormon- und /oder Neurotransmitter-Ungleichgewichten des neuroendokrinen Regelkreises einhergehen, geeignet sind.

Die Erfindung soll anhand von Ausführungsbeispielen erläutert werden, ohne auf diese Beispiele beschränkt zu sein.

### Ausführungsbeispiele

Die nachfolgend aufgeführte Übersicht zeigt eine schematische Darstellung für Behandlungskonzepte bei neuroendokrinen Störungen mit Hilfe der erfindungsgemäßen Nahrungsergänzungsmittel (NEM):

Beispiel 0: Behandlungskonzept neuroendokriner Störungen

**Behandlungskonzept neuroendokriner Störungen**

| | **Initialtherapie** | | **Restauration** | |
|---|---|---|---|---|
| Diagnose | morgens | abends | morgens | abends |
| Chronische Stressbelastung | NEM1/1A | NEM8/8A | NEM1/1A | NEM1/1A |
| | | | NEM7/7A | |
| Burn-Out-Syndrom | NEM1/1A | NEM7/7A | NEM1/1A | NEM1/1A |
| | | | NEM7/7A | |
| Müdigkeit /Fatigue | NEM4/4A | NEM5/5A | NEM1/1A | NEM1/1A |
| | | | NEM7/7A | |
| Antriebsschwäche | NEM4/4A | | NEM4/4A | |
| | | | NEM7/7A | |
| Schlafstörungen | NEM8/8A | NEM5/5A | NEM7/7A | NEM1/1A |
| Ängste | NEM8/8A | NEM5/5A | NEM7/7A | NEM5/5A |
| Panikattacken | NEM8/8A | NEM5/5A | NEM7/7A | |
| MCS | NEM7/7A | NEM5/5A | NEM7/7A | NEM5/5A |
| CFS | NEM8/8A | NEM5/5A | NEM1/1A | NEM5/5A |
| | NEM1/1A | | NEM7/7A | |
| Fibromyalgie | NEM8/8A | | NEM7/7A | NEM7/7A |
| Depressionen primär | NEM8/8A | | NEM1/1A | |
| | NEM7/7A | | NEM7/7A | |
| Depressionen reaktiv | NEM8/8A | NEM5/5A | NEM1/1A | NEM5/5A |
| | NEM1/1A | | NEM7/7A | |
| Kopfschmerzen | NEM8/8A | NEM3/3A | NEM8/8A | NEM3/3A |
| Migräne | NEM8/8A | NEM7/7A | NEM7/7A | NEM5/5A |
| PMS prämenstruelles Syndrom | NEM7/7A | NEM8/8A | NEM7/7A | NEM7/7A |
| Menopause-Beschwerden | NEM8/8A | | NEM7/7A | NEM7/7A |
| Heißhunger | NEM6/6A | NEM3/3A | NEM7/7A | NEM6/6A |
| Übergewicht /Adipositas | NEM7/7A | NEM6/6A | NEM7/7A | NEM3/3A |
| Reizdarmsyndrom | | NEM8/8A | | NEM7/7A |
| ADS/ADHS | NEM2/2A | NEM8/8A | NEM7/7A | |
| Autismus | | NEM2/2A | NEM1/1A | NEM7/7A |

### Beispiel 1:

Nahrungsergänzungsmittel 1A (NEM1A):

### Inhaltsstoffe pro Tagesdosis = 2 Kaps.:

| | | | |
|---|---|---|---|
| Vitamin B5 | 100,1 mg | Vitamin C | 100 mg |
| Vitamin B6 | 4,1 mg | Vitamin B3 | 2 mg |
| Vitamin B 12 | 20 µg | Folsäure | 80 µg |
| Magnesium | 130 mg | Zink | 1 mg |
| L-Glutamin | 150 mg | L-Tyrosin | 150 mg |
| L-Histidin | 80 mg | Rhodiola rosea | 30 mg |
| Grün-Tee-Extrakt | 36 mg | | |

### Beispiel 2:

Nahrungsergänzungsmittel 2A (NEM2A):

### Inhaltsstoffe pro Tagesdosis = 2 Kaps.:

| | | | |
|---|---|---|---|
| Vitamin B6 | 10,2 mg | Folsäure | 100 µg |
| Vitamin B12 | 5 µg | Magnesium | 13,5 mg |
| Rhodiola rosea | 200 mg | Taurin | 130 mg |
| Phosphatidylcholin | 90 mg | Theanin | 75 mg |
| HTP | 25 mg | Mucuna pruriens | 12,5mg |

### Beispiel 3:

Nahrungsergänzungsmittel 3A (NEM3A):

### Inhaltsstoffe pro Tagesdosis = 2 Kaps.:

| | | | |
|---|---|---|---|
| Vitamin C | 60 mg | Vitamin B6 | 12,7 mg |
| Folsäure | 67 µg | Calcium | 37 mg |
| Selen | 371 µg | N-Acetyl-Cystein | 425 mg |
| L-Tyrosin | 100 mg | Mucuna pruriens | 100mg |

### Beispiel 4:

Nahrungsergänzungsmittel 4A (NEM4A):

### Inhaltsstoffe pro Tagesdosis = 2 Kaps.:

| | | | |
|---|---|---|---|
| Vitamin C | 30 mg | Vitamin B5 | 20,1 mg |
| Vitamin B6 | 8,4 mg | Vitamin B3 | 5 mg |
| Folsäure | 67 µg | Zink | 3,3 mg |
| Mangan | 1,3 mg | L-Tyrosin | 166,8 mg |
| L-Histidin | 150 mg | N-Acetyl-Cystein | 66,8 mg |
| Rhodiola rosea | 36 mg | Mucuna pruriens | 33,4 mg |

### Beispiel 5:

Nahrungsergänzungsmittel 5A (NEM5A):

| | | | |
|---|---|---|---|
| Vitamin C | 40 mg | Vitamin B6 | 12,4 mg |
| Folsäure | 68 µg | Magnesium | 50 mg |
| L-Glutamin | 350 mg | Taurin | 250 mg |
| Theanin | 75 mg | HTP | 50 mg |
| L-Tyrosin | 50 mg | Tocopherole gemischt | 15 mg |

### Beispiel 6:

Nahrungsergänzungsmittel 6A (NEM6A):

### Inhaltsstoffe pro Tagesdosis = 2 Kaps.:

| | | | |
|---|---|---|---|
| Vitamin B3 | 50 mg | Vitamin B5 | 30 mg |
| Vitamin B6 | 6,7 mg | Folsäure | 44 µg |
| Vitamin B12 | 43 µg | Zink | 42,7 mg |
| Chrom | 67 µg | Selen | 19 µg |
| Mangan | 10 µg | N-Acetyl-Cystein | 100 mg |
| Grün-Tee-Extrakt | 60 mg | Mucuna pruriens | 33,4 mg |
| HTP | 25 mg | | |

### Beispiel 7:

Nahrungsergänzungsmittel 7A (NEM7A):

### Inhaltsstoffe pro Tagesdosis = 4 Kaps.:

| | | | |
|---|---|---|---|
| Vitamin C | 80 mg | Vitamin B6 | 40 mg |
| Folsäure | 268 µg | Calcium | 120 mg |
| L-Tyrosin | 1200 mg | HTP | 200 mg |
| Theanin | 136 mg | L-Phenylalanin | 100 mg |

### Beispiel 8:

Nahrungsergänzungsmittel 8A (NEM8A):

### Inhaltsstoffe pro Tagesdosis = 2 Kaps.:

| | | | |
|---|---|---|---|
| Vitamin C | 20 mg | Vitamin B6 | 8,4 mg |
| Folsäure | 68 µg | Vitamin B 12 | 10 µg |
| Magnesium | 66,7 mg | Zink | 3,3 mg |
| Selen | 232 µg | Taurin | 150 mg |
| N-Acetyl-Cystein | 150 mg | HTP | 100 mg |
| Theanin | 50 mg. | | |

### Abkürzungsverzeichnis

- AD: Alzheimer's Desease / Alzheimer-Krankheit
- ADS: Aufmerksamkeits-Defizit-Syndrom
- ADHS: Aufmerksamkeits-Defizit-Hyperaktivitäts-Syndrom (ADS mit Hyperaktivität)
- CA: Kanadische Patentschrift
- CFIDS: Chronisches Müdigkeitssyndrom (CFS bzw. CFIDS) Chronic Fatigue Immunodysfunction Syndrome
- CFS: Chronisches Müdigkeitssyndrom (Chronic Fatigue Syndrom)
- CMI: Chronic Multisystem Illnesses
- CRH: Corticotropin-Relasing Hormon (ein Neurostresshormon)
- DE-OS: Deutsche Offenlegungsschrift
- DHEA: Dehydroepiandrosteron
- EGCG: Epigallocatechin-3-gallat (Grüntee)
- EGCG: Grüntee (EGCG)
- EP: Europäische Patentschrift
- FMS: Fibromyalgie-Syndrom
- GABA: Gamma-Aminobuttersäure
- MCS: Multiple Chemikalienüberempfindlichkeit (multiple chemical sensitivity)
- NEM: Nahrungsergänzungsmittel
- HTP: Hydroxytryptophan
- 5-HTP: 5-Hydroxytryptophan
- OS: Offenlegungsschrift
- OTS: Overtrained Athlete Syndrome, Übertrainingssyndrom
- PEA: Phenylethylamin
- PMS: Prämenstruelles Syndrom
- PTSD: Post traumatic Stress Disorder, Posttraumatisches Stresssyndrom
- SAD: Saisonal Affective Disorder, Winterdepression
- SBS: Sick Building Syndrom
- WIPO: Weltorganisation für Geistiges Eigentum
- WIPO: World Intellectual Property Organization
- WO: Internationale Patentschrift (der WIPO)

## Patentansprüche

1. Verwendung von Neurotransmitter-Derivaten - Aminosäurevorstufen von Neurotransmittern, Kofaktoren für die Synthese von Neurotransmittern und Neurotransmitter-Modulatoren - zur Herstellung von Mitteln zur Behandlung von neuroendokrinen gesundheitlichen Störungen, die mit Stresshormon- und /oder Neurotransmitter-Ungleichgewichten des neuroendokrinen Regelkreises einhergehen.

2. Verwendung nach Anspruch 1, **dadurch gekennzeichnet, dass**
2.1. die Aminosäurevorstufen aus Tyrosin und /oder Acetyltyrosin und /oder Phenylalanin und /oder 5-Hydroxytryptophan (5-HTP) und /oder Mucuna und /oder Glutamin und /oder Histidin
2.2. die Neurotransmitter-Modulatoren aus Taurin und /oder Theanin und /oder Rhodiola rosea und /oder Grüntee (EGCG) und /oder aus Phosphatidylcholin und /oder Phosphatidylserin
2.3. die Kofaktoren für die Synthese von Neurotransmittern aus den Vitaminen C und /oder B3, B5, B6, B12 und /oder Vitamin E (Tocopherole) und /oder N-Acetylcystein und /oder Folat und /oder aus Magnesium-, Calcium-, Selen-, Zink-, Mangan- und /oder Chrom-Verbindungen
bestehen.

3. Verwendung nach Anspruch 1 und 2, **dadurch gekennzeichnet, dass** die Mittel zur Behandlung von neuroendokrinen gesundheitlichen Störungen, von Neurostresszuständen, von Neuroregulationsstörungen oder von Regulationsstörungen der neuroendokrinen Stressachse der Art
a) ADS /ADHS (bei Kindern, Erwachsenen)
b) CFS /Chronisches Müdigkeitssyndrom
c) Fatigue (Müdigkeit /Erschöpfbarkeit)
d) MCS /Multiple Chemische Sensitivität
e) Fibromyalgie
f) Kopfschmerzen /Migräne
g) Übergewicht /Adipositas
h) Kohlehydrat-Heißhunger
i) Appetitstörungen
j) Depressionen
j1) primäre, endogene Depressionen / rezidivierende depressive Episoden
j2) sekundäre, reaktive oder somatogene Depressionen / depressive Episoden
j3) Stress-Depressionen
j4) Winter- /Frühjahrsdepressionen
j5) Depressionen infolge Pharmakotherapie (Zytokine, Zytostatika)
k) Angstsyndromen
k1) Panikattacken
k2) Klaustrophobie
l) Schlafstörungen
m) Nahrungsmittelunverträglichkeiten
n) Prämenstruelles Syndrom
o) Prä- /Perimenopause-Beschwerden
p) Irritables Kolon (Reizdarm)
q) Burn-Out-Syndrom
r) Sickness-Syndrom
s) Muskelschmerzen
t) Konzentrationsstörungen
u) Gedächtnisschwäche (Kurzzeitgedächtnis)
v) Koordinationsstörungen
w) CMI (Chronic Multisystem Illnesses)
x) Sick-Building Syndrome (SBS)
y) Building related illness
z) Environmental somatisation syndrome
α) Idiopatic Environmental Intolerance
β) Übertrainingssyndrom (OTS: Overtrained Athlete Syndrome)
γ) SAD / Saisonal Affective Disorder
δ) Übertrainingssyndrom (OTS: Overtrained Athlete Syndrome)
ε) Chronischer Stress
ζ) Posttraumatisches Stresssyndrom (PTSD: Post traumatic Stress Disorder)
η) Autismus
dienen.

4. Verwendung nach Anspruch 1 bis 5, **dadurch gekennzeichnet, dass** die Mittel Nahrungsergänzungsmittel (NEM) darstellen, die gemeinsam Aminosäurevorstufen von Neurotransmittern, Neurotransmitter-Modulatoren, Kofaktoren für die Synthese von Neurotransmittern, Vitamin C und Vitamine der Reihe B enthalten.

5. Nahrungsergänzungsmittel NEM1 bis NEM8 nach Anspruch 4, umfassend
5.1. L-Glutamin, L-Tyrosin und L-Histidin als wesentliche Komponenten in NEM1
5.1.1. als NEM1A - zusätzlich zu NEM -Vitamin B5 und /oder Vitamin C, Vitamin B6, Vitamin B3, Vitamin B12, Folsäure, Magnesium, Zink, Rhodiola rosea und /oder Grün-Tee-Extrakt
5.2. Theanin, 5-HTP und Mucuna pruriens als wesentliche Komponenten in NEM2
5.2.1. als NEM2A - zusätzlich zu NEM2 -Vitamin B6 und /oder Folsäure, Vitamin B12, Magnesium, Rhodiola rosea, Taurin und /oder Phosphatidylcholin
5.3. L-Tyrosin und Mucuna pruriens als wesentliche Komponenten in NEM3
5.3.1. als NEM3A - zusätzlich zu NEM3 -Vitamin C und /oder Vitamin B6, Folsäure, Calcium, Selen und /oder N-Acetyl-Cystein
5.4. L-Tyrosin, L-Histidin und Mucuna pruriens als wesentliche Komponenten in NEM4
5.4.1. als NEM4A - zusätzlich zu NEM4 -Vitamin C und /oder Vitamin B5, Vitamin B6, Vitamin B3, Folsäure, Magnesium, Zink, Mangan, N-Acetyl-Cystein und /oder Rhodiola rosea
5.5. L-Glutamin, Theanin, 5-HTP und L-Tyrosin als wesentliche Komponenten in NEM5
5.5.1. als NEM5A - zusätzlich zu NEM5 -Vitamin C und /oder Vitamin B6, Folsäure, Magnesium, Taurin, und /oder Tocopherolen /Tocotrienolen
5.6. Mucuna pruriens und 5-HTP als wesentliche Komponenten in NEM6
5.6.1. als NEM6A - zusätzlich zu NEM5 -Vitamin B3 und /oder Vitamin B5, Vitamin B6, Folsäure, Vitamin B12, Zink, Chrom, Selen, Mangan, N-Acetyl-Cystein und /oder Grün-Tee-Extrakt
5.7. L-Tyrosin, 5-HTP und Theanin als wesentliche Komponenten in NEM7
5.7.1. als NEM7A - zusätzlich zu NEM7 -Vitamin C und /oder Vitamin B6, Folsäure, Calcium und /oder L-Phenylalanin
5.8. 5-HTP und Theanin als wesentliche Komponenten in NEM8
5.8.1. als NEM8A - zusätzlich zu NEM8 -Vitamin C und /oder Vitamin B6, Folsäure, Vitamin B12, Magnesium, Zink, Selen, Taurin und /oder N-Acetyl-Cystein.

6. Verwendung nach Anspruch 1 bis 4 und NEM nach Anspruch 5, **dadurch gekennzeichnet, dass** die basischen Verbindungen der Mittel und der NEM auch in Form ihrer physiologisch verträglichen Salze vorliegen.

7. Verwendung nach Anspruch 1 bis 4 und NEM nach Anspruch 5 und 6, **dadurch gekennzeichnet, dass** die Mittel und die NEM zur Initial- und Restaurations-Behandlung neuroendokriner Störungen mit Hilfe der erfindungsgemäßen Nahrungsergänzungsmittel (NEM) nach Anspruch 4 bis 6 für die nachfolgend genannten Erkrankungen dienen:
| | **Initialtherapie** | | **Restauration** | |
|---|---|---|---|---|
| Diagnose | morgens | abends | morgens | abends |
| Chronische Stressbelastung | NEM1/1A | NEM8/8A | NEM1/1A NEM7/7A | NEM1/1A |
| Burn-Out-Syndrom | NEM1/1A | NEM7/7A | NEM1/1A NEM7/7A | NEM1/1A |
| Müdigkeit /Fatigue | NEM4/4A | NEM5/5A | NEM1/1A NEM7/7A | NEM1/1A |
| Antriebsschwäche | NEM4/4A | | NEM4/4A NEM7/7A | |
| Schlafstörungen | NEM8/8A | NEM5/5A | NEM7/7A | NEM1/1A |
| Ängste | NEM8/8A | NEM5/5A | NEM7/7A | NEM5/5A |
| Panikattacken | NEM8/8A | NEM5/5A | NEM7/7A | |
| MCS | NEM7/7A | NEM5/5A | NEM7/7A | NEM5/5A |
| CFS | NEM8/8A NEM1/1A | NEM5/5A | NEM1/1A NEM7/7A | NEM5/5A |
| Fibromyalgie | NEM8/8A | | NEM7/7A | NEM7/7A |
| Depressionen primär | NEM8/8A NEM7/7A | | NEM1/1A NEM7/7A | |
| Depressionen reaktiv | NEM8/8A NEM1/1A | NEM5/5A | NEM1/1A NEM7/7A | NEM5/5A |
| Kopfschmerzen | NEM8/8A | NEM3/3A | NEM8/8A | NEM3/3A |
| Migräne | NEM8/8A | NEM7/7A | NEM7/7A | NEM5/5A |
| PMS prämenstruelles Syndrom | NEM7/7A | NEM8/8A | NEM7/7A | NEM7/7A |
| Menopause-Beschwerden | NEM8/8A | | NEM7/7A | NEM7/7A |
| Heißhunger | NEM6/6A | NEM3/3A | NEM7/7A | NEM6/6A |
| Übergewicht /Adipositas | NEM7/7A | NEM6/6A | NEM7/7A | NEM3/3A |
| Reizdarmsyndrom | | NEM8/8A | | NEM7/7A |
| ADS/ADHS | NEM2/2A | NEM8/8A | NEM7/7A | |
| Autismus | | NEM2/2A | NEM1/1A | NEM7/7A |
